# EUROPEAN PATENT SPECIFICATION

(11) **EP 3 813 843 B1**
(45) Date of publication and mention of the grant of the patent: **06.09.2023**
(21) Application number: 19806044.4
(22) Date of filing: 25.06.2019
(51) Int. Cl.: A61K 31/704, A61P 29/00, A61K 36/324, A61K 36/77, A61K 36/539

(54) **COMPOSITION FOR USE IN THE PREVENTION AND/OR TREATMENT OF OSTEOARTICULAR DISEASES**
ZUSAMMENSETZUNG ZUR VERWENDUNG BEI DER VORBEUGUNG UND/ODER BEHANDLUNG VON KNOCHEN-GELENK-ERKRANKUNGEN
COMPOSITION DESTINÉE À ÊTRE UTILISÉE DANS LA PRÉVENTION ET/OU LE TRAITEMENT DE MALADIES OSTÉOARTICULAIRES

(30) Priority: 26.06.2018 IT 201800006683
(43) Date of publication of application: 05.05.2021
(73) Proprietor: Neilos S.r.l., 80063 Piano di Sorrento (NA) (IT)
(72) Inventor: DI MAIO, Umberto, 80065 Sant' Agnello (Napoli) (IT)
(74) Representative: Comoglio, Elena
(86) International application number: PCT/IB2019/055322
(87) International publication number: WO 2020/003106

(56) References cited:
- EP-A1- 2 149 378
- WO-A1-2004/058279
- MELAINIE CAMERON ET AL: "Oral herbal therapies for treating osteoarthritis", COCHRANE DATABASE OF SYSTEMATIC REVIEWS, 22 May 2014 (2014-05-22), XP055560554, DOI: 10.1002/14651858.CD002947.pub2

## Description

The present invention concerns a composition of substances, preferably obtained from natural sources, effective in the prevention and/or therapeutic treatment of osteoarticular diseases, particularly arthritis.

The term "arthritis" refers to all disorders affecting the joints. In all cases the patients are affected by pain and stiffness of the joints. There are about 100 different types of arthritis of which the most common are rheumatoid arthritis and osteoarthritis.

Rheumatoid arthritis is an autoimmune disorder that mainly involves the joints, which become swollen and painful. The disease may also affect other parts of the body, leading for example to a reduction in the number of red blood cells, the appearance of inflammation around the heart and lungs, fever and a general sense of fatigue.

It is believed that this disease is due to a combination of genetic and environmental factors. The main mechanism involves the activation of the immune system against the joints, which leads to inflammation and thickening of the articular capsule, also involving the bone and cartilage. The initial site of the disease is the synovial membrane, where there is an infiltration of cells of the immune system. More specifically, rheumatoid arthritis is generally characterized by the succession of three phases: an initial phase due to non-specific inflammation, an amplification phase involving the activation of T cells, and finally a final phase of chronic inflammation due to the release of pro-inflammatory cytokines, such as interleukin-2, γ-interferon, tumor necrosis factor (TNF) and interleukin-6.

The main risk factors for rheumatoid arthritis are gene mutations that involve disorders in the regulation of the immune response. One of the gene mutations responsible for the etiology of rheumatoid arthritis is localized in genes involved in the expression of the class 2 major histocompatibility complex (MHC) such as HLA DR4. Other documented environmental factors include, for example, smoking.

Osteoarthritis (osteoarthrosis or arthrosis) is a degenerative disease of the joints that affects millions of people around the world mainly in the hips, hands and knees. It is well known that the synovia, bone and cartilage are the tissues most involved in the pathological mechanisms of osteoarthritis.

The cause of the disease is likely to be related to changes in the homeostasis of the joint cartilage and bone that lead to the increase of destructive processes. In osteoarthritis there is a change in the structure of the bone and a degeneration of the joint cartilage. The subchondral bone becomes rigid, less capable of absorbing impact loads, which leads to greater stress on the cartilage. In fact, the main characteristics of the disease include a progressive loss of cartilage tissue, hypertrophic bone modifications and formation of osteophytes that grow at the edges of the bones involved.

With reference to cartilage, it is known that chondrocytes are the cells responsible for balancing the processes of synthesis and destruction of the matrix, capable of regulating cytokines and growth factors. In degenerative osteoarticular processes, this balance is usually compromised. In patients with osteoarthritis, chondrocytes produce high levels of inflammatory cytokines such as interleukin 1-β and tumor necrosis factor (TNF), which in turn reduce collagen synthesis and increase concentrations of catabolic mediators such as metalloproteases. At the same time, other pro-inflammatory substances such as interleukin-8 (IL-8) and interleukin-6 (IL-6), prostaglandin E2 and nitric oxide are released. The increase of oxidizing agents such as nitric oxide determines the apoptosis of chondrocytes and thus the degeneration of the matrix.

In addition to the inflammatory process, another event typically found in osteoarthritis is a progressive reduction in the level of proteoglycans. Without the protective effect of proteoglycans, the cartilage becomes much more susceptible to degeneration and degradation. Aggrecan is considered the main proteoglycan responsible for providing hydration and elasticity to cartilage tissues, and, in patients with osteoarthritis, a reduction in its concentration is observed.

Moreover, several studies have shown that chondrocytes are also responsible for the production of reactive oxygen species (ROS), such as superoxide anions (O²⁻) and hydroxyl radicals (OH⁻). This effect is mainly due to the activation of macrophages and neutrophils, which take part in the inflammatory response.

The most documented risk factors for osteoarthritis are age, sex, obesity, genetics and trauma which may determine the onset of the physiopathology of osteoarthritis. Age is the main risk factor, as it is known that with increasing age the tensile properties of the articular cartilage decrease, leading to mechanical problems. Women normally show greater pain and disability than men. People with a high body mass index are more at risk.

The most common symptoms of osteoarthritis include chronic pain, due to an increased concentration at the affected sites of excitatory amino acids such as glutamate, which contributes to hyperalgesia and pain. Another characteristic symptom is articular rigidity due to a decrease in the levels of surfactant phospholipids responsible for reducing friction ensuring optimal lubrication.

Due to changes in, for example, the articular cartilage in the knee, additional effects appear in patients, including edema of the underlying soft tissues, blood circulation disorders, bone enlargement and swelling.

The phannacological treatments traditionally used in osteoarticular diseases are primarily represented by analgesic drugs, which help to reduce the sensation of pain but, obviously, do not represent anything but symptomatic treatments. Anti-inflammatory drugs of various kinds (nonsteroidal and steroidal) are also used to counteract the inflammatory processes that characterize the disease. However, these drugs require numerous daily administrations to reach adequate therapeutic doses and often have serious side effects, such as peptic ulcers or gastric perforations. The use of selective COX-2 NSAIDs (such as Celecoxib) reduces this type of event, but at the same time may cause serious damage to the cardiovascular system. Paracetamol has fewer side effects but is only indicated in mild to moderate forms of osteoarthritis. EP2149378 A1 discloses a composition comprising capsaicin, boswellic acids and escin for use in treating osteoarthritis.

There is therefore a need to provide treatments, alternative to those already existing, that are effective in the prevention and/or therapeutic treatment of osteoarticular diseases, but that do not have the side effects and/or disadvantages of treatments in the state of the art.

These and other needs are met by the present invention, which provides a composition characterized in that it comprises a synergistic combination of active ingredients, obtained from natural sources, the aforesaid combination having proved particularly effective against osteoarticular diseases.

The composition of the invention is as defined in the accompanying claim 1. Further features and advantages of the invention are defined in the dependent claims. The claims form an integral part of the present description.

Hereinafter a detailed description of some of the preferred embodiments of the invention is provided.

The synergistic composition of the present invention is useful for the treatment and prevention of osteoarticular diseases.

In the composition of the present invention, the synergistic action takes place between aescin, at least one extract of a plant belonging to the genus *Boswellia,* preferably at least one extract of *Boswellia serrata,* and an extract of *Scutellaria baicalensis.*

*Boswellia serrata* belongs to the family *Burseraceae* and to the genus *Boswellia* and is a tree of moderate size that grows in the mountainous areas of India, North Africa and Middle East. The most interesting part of the plant from a pharmacological point of view is the gum-resin or frankincense that is obtained by an incision of the trunk. This oily resin contains about 30-60% resin, 5-10% essential oil, and the remaining part polysaccharides, such as arabinose, galactose, xylose. Due to the essential oil, the resin has a particular aroma that justifies its use in aromatherapy. The resin also contains monoterpenes (α-thujene), diterpenes (macrocyclic diterpenes such as incensole, incensole oxide, iso incensole oxide, serratol), triterpenes (such as α and β amyrin), pentacyclic triterpene acids (boswellic acids) and tetracyclic triterpene acids.

Boswellic acids are the main secondary metabolites of frankincense with anti-inflammatory and anti-tumor activity. Clinical studies have shown the effectiveness of *Boswellia*-based preparations in the treatment of inflammatory diseases, such as osteoarthritis, rheumatoid arthritis, chronic inflammatory intestinal diseases, as well as in the treatment of asthma and cancer.

Several mechanisms have been proposed for the action of boswellic acids on inflammation. For example, 3-O-Acetyl-11-ketoboswellic acid is able to interfere with several pathways, including the NF-κB pathway and the Mitogen-Activated Protein kinase (MAP kinase) pathway. In addition, the ability of boswellic acids to interact with 5-lipoxygenase (5-LOX) and cyclooxygenase isoform 1 (COX-1) has been demonstrated.

A potentially interesting action mechanism is the inhibition of microsomal prostaglandin E2 synthase type 1 (mPGES-1). PGES, prostaglandin E synthase, is an enzyme involved in the last step of the biosynthetic pathway of PGE2, which consists in the conversion of PGH2, the synthesis of which is catalyzed by cyclooxygenase, into PGE2. It has been shown that the mPGES-1 isoform may be induced by pro-inflammatory stimuli, such as interleukin-1β (IL-1β) and lipopolysaccharide (LPS). In addition, mPGES-1 receives prostaglandin H2 preferentially from the COX-2 enzyme, since the two enzymes are co-expressed and colocated and their expression may be induced by pro-inflammatory stimuli. Inflammation, pain and fever may be induced by PGE2 derived from over-expression of this enzyme. Inhibition of mPGES-1 may be effective in the treatment of inflammatory diseases, prostaglandin E2-induced hyperalgesia and fever. All boswellic acids have good inhibitory activity against mPGES-1, however β-boswellic acid has been shown to be one of the most active, with an IC₅₀ of 10 µM in intact A549 cell assays. Assays *in vivo* on acute inflammation models (carrageenan edema and pleurisy) have shown good activity at doses of 1 mg/kg, comparable to a dose of 5 mg/kg of indomethacin.

Boswellic acids are also able to limit *in vivo* the degradation of glycosaminoglycans induced by treatment with NSAIDs (ketoprofen), which accelerates joint damage in situations of arthritis.

In a clinical study carried out on 30 patients with osteoarthritis of the knee, the administration of 1 g of *Boswellia* per day was shown to reduce pain and swelling and also increase mobility compared to the placebo. In combination with curcumin, Indian ginseng and zinc, *Boswellia* extract has been shown to reduce pain severity and disability in patients with osteoarthritis. Finally, in a comparative study with a placebo, *Boswellia* intake reduced the intake of NSAIDs during treatment in patients with rheumatoid arthritis by about twice as much as the placebo.

*Aesculus Hippocastanum L.* is a large tree known as the horse-chestnut or conker tree, belonging to the *Hippocastanaceae* family. The drug consists of dried seeds, which contain no less than 3% of triterpene glycosides. The main chemical constituents characteristic of the drug are collectively known as aescin, a name that represents a mixture of acrylated triterpene glycosides (saponins), the aglycones of which are mainly protoaescigenin and barringtogenol C. The difference between these aglycones is due to the presence of hydroxyl in the C-24 position of the protoaescigenin molecule. All the saponins of the horse-chestnut have a trisaccharide group bound in the C-3 position consisting of glucuronic acid which may be combined with glucose, galactose or xylose. The two main saponins, both derived from protoaescigenin, are esterified in position 21β with angelic acid and in position 22α with acetic acid. To date, 30 different saponins contained in aescin have been identified, which may be grouped in three fractions: β-aescin, which contains only 22-O-acetyl compounds; cryptoaescin, which contains only 28-O-acetyl compounds; α-aescin, which is a mixture of β-aescin and cryptoaescin. Other drug constituents include flavonoids (di- and triglycosides of quercetin and kaempferol, which make up 0.3% of the drug), sterols, an essential oil and a significant amount of starch.

Aescin and the saponin fractions of the drug exert an anti-inflammatory activity, demonstrated in several animal experiments: inhibition of the edema induced by albumin or carrageenan in the rat's paw or inhibition of exudative edema induced by dextran in the rat's skin; the mechanism on which this activity depends seems to be the inhibition of the prostaglandin synthetase enzyme, as shown by an *in vitro* experiment with a saponin fraction. According to the scientific literature, aescin is able to act at different levels in the pathway of glucocorticoids by stimulating the release thereof or increasing the expression of receptors with consequent stimulation of the anti-inflammatory effect. In a study on rats with induced arthritis, the combination of prednisone (2 mg/kg/day) and aescin (5 or 10 mg/kg/day) proved effective with synergistic action in reducing the paw edema by reducing the expression of cytokines such as TNF-α, interleukin 1-β and interleukin 6.

In a different experiment, a saponin fraction (7.5 mg/kg) administered to the rat orally was shown to exert significant analgesic activity. The clinical effects of the saponin fractions of the drug have been evaluated in several trials. In placebo-controlled crossover studies conducted on women suffering mainly from varicose veins and chronic venous insufficiency, oral administration twice daily for 20 days of a 300 mg dose of a saponin fraction (corresponding to 50 mg aescin) resulted in a significant reduction of various leg symptoms: edema, inflammation, pain, and also itching, pressure sensitivity, pigmentation, fatigue, heaviness and cramp. In a randomized, double-blind, placebo-controlled study, the anti-edema effect of a saponin fraction administered for 6 weeks to 39 patients at a daily dose corresponding to 150 mg of aescin was tested. It may therefore be deduced that the ideal daily dose of triterpene glycosides calculated as aescin is between 50 and 150 mg/day.

*Scutellaria baicalensis* is a plant belonging to the family *Lamiaceae* cultivated in China, Japan, Korea. In these countries, the monograph of the plant is included in the official pharmacopoeia.

The root extracts are particularly rich in flavonoids which are responsible for the characteristic yellow color. Many biological properties have been described for this plant: anti-inflammatory, antiviral, anti-tumor, antioxidant and immunostimulant.

In traditional medicine the plant has been used to treat allergic conditions, inflammatory gastrointestinal viral infections, respiratory infections, bile infections, etc.

The flavonoids present in the plant are probably the most active components from a pharmacological point of view. Several studies in the literature demonstrate the anti-inflammatory efficacy of baicalin, which may reduce levels of NF-κB and COX-2 in rats while increasing levels of superoxide dismutase. This compound is also able to act at the level of INOS causing an inhibition of this enzyme involved in the synthesis of nitric oxide and vasodilatation typical of inflammatory processes.

In the treatment of osteoarthritis, *Scutellaria baicalensis* extract, together with an *Acacia catechu* extract, has been shown to inhibit the activity of COX 1 and 2 and 5-LOX in cellular and animal models.

The therapeutic efficacy of the association of *Scutellaria baicalensis* and *A. catechu* has also been assessed on men with osteoarthritis of the knee. In this study, patients received a combination of the two extracts (500 mg) or naproxen (440 mg) for a treatment period of one week. The study has demonstrated that the combination of the two extracts is able to reduce pain perception and stiffness in a manner comparable to naproxen and improve joint flexibility. The safety profile and tolerability of the combination of *Scutellaria* extract with *Acacia catechu* extract have been assessed in 59 patients with osteoarthritis of the knee, administering a total of 500 mg/day for 12 weeks. The preparation has proven to be safe and with minor side effects compared to the placebo, wherein the respiratory side effects were actually greater (scutellaria 2).

The anti-inflammatory activity exerted by the flavonoids present in the *Scutellaria baicalensis* extracts is of particular interest in the field of application of the present invention; in fact, the administration of this extract could ensure a reduction in the symptoms of osteoarthritis with a good profile of safety and tolerability.

As indicated previously, the synergistic composition of the present invention is effective in the treatment and/or prevention of osteoarticular diseases. To summarize, the effectiveness of the composition derives from the following activities of the components:
- Extracts of plants belonging to the genus *Boswellia* and in particular of *Boswellia serrata* are inhibitors of 5-LOX and mPGES-1 with anti-inflammatory and analgesic activity;
- Aescin, preferably obtained from an extract of *Aesculus hippocastanum,* has an anti-edema and anti-inflammatory action;
- Due to its flavonoids, *Scutellaria baicalensis* extract is able to inhibit NF-κB and COX-2 by also increasing the superoxide dismutase levels.

As discussed previously, in the present invention, the synergistic action takes place between aescin, at least one extract of a plant belonging to the genus *Boswellia,* preferably at least one extract of *Boswellia serrata,* and an extract of *Scutellaria baicalensis.*

In a preferred embodiment, aescin is administered in a daily dose of between 1 mg and 5000 mg, for example by administering a dosage form wherein aescin is present in a quantity of between 0.5% and 70% w/w, more preferably in a quantity of between 2 and 50% by weight of the total weight of the composition; at least one extract of a plant belonging to the genus *Boswellia,* preferably at least one extract of *Boswellia serrata,* is administered in a daily dose of between 10 mg and 5000 mg, for example by administration of a dosage form wherein the aforesaid at least one extract is present in a quantity of between 1 and 90% w/w, more preferably in a quantity of between 5 and 80% by weight of the total weight of the composition; *Scutellaria baicalensis* extract is administered in a daily dose of between 1 mg and 5000 mg, for example by administration of a dosage form wherein the aforesaid extract is present in a quantity of between 0.5 and 70% w/w, more preferably in a quantity of between 2 and 50% by weight of the total weight of the composition.

The dosage form may be a pharmaceutical composition or a food supplement or a medical device or a food for special medical purposes including the aforementioned active ingredients in a mixture. The preferred route of administration is oral or topical.

The following examples are provided purely for illustrative purposes and are not intended to limit the scope of the invention as defined in the accompanying claims.

### EXAMPLES

The following examples concern any form of pharmaceutical dosage that is acceptable, preferably oral or topical, suitable in the scope of the present invention.

Some examples of formulations are given, with the quantities of the relative active substances present in each dosage unit.

### EXAMPLE 1

| Active ingredient | Daily dose |
|---|---|
| *Boswellia serrata* e.s. | 1 g |
| Aescin from *Aesculus Hippocastanum* | 50 mg |
| *Scutellaria baicalensis* e.s. | 250 mg |

### EXAMPLE 2

| Active ingredient | Daily dose |
|---|---|
| *Boswellia serrata* e.s. | 500 mg |
| Aescin from *Aesculus Hippocastanum* | 100 mg |
| *Scutellaria baicalensis* e.s. | 50 mg |

### EXAMPLE 3

| Active ingredient | Daily dose |
|---|---|
| *Boswellia serrata* e.s. | 1200 mg |
| Aescin from *Aesculus Hippocastanum* | 40 mg |
| *Scutellaria baicalensis e.s.* | 50 mg |

### EXAMPLE 4

| Active ingredient | Daily dose |
|---|---|
| *Boswellia serrata* e.s. | 350 mg |
| Aescin from *Aesculus Hippocastanum* | 20 mg |
| *Scutellaria baicalensis* e.s. | 150 mg |

### EXPERIMENTAL PART

The synergistic action of the composition with respect to the individual active ingredients is evaluated using experimental methods *in vitro* and/or *in vivo.*

Various cell studies are able to provide important information about the anti-inflammatory capacity of bioactive compounds able to inhibit cytokine expression. IC50 studies are able to provide information for cellular inhibition. To evaluate in more detail the response (inhibition/activation) of cytokines (TNF-α, IL-1, IL-4, IL-6, IL-8, IL-10,IL-17, INF-Y, COMP) during inflammatory processes induced by LPS, the "Whole Blood Assay (WBA)" or the "peripheral blood mononuclear cell" (PBMC) test or other similar tests are used.

As an *in vivo* test, a simple model of general inflammation is the model of carrageenan-induced edema in mice or rats. In this test, the injection of carrageenan into the animal's paw results in an acute inflammatory reaction of the paw with the appearance of classic signs of inflammation; this reaction reaches its maximum within 3 hours after inoculation. This test may be used to assess the ability of the active ingredients of the present invention to reduce the edema of the paw induced by the injection of carrageenan.

More complex methods that are used for the evaluation of osteoarticular diseases are for example the model of arthritis induced by collagen and arthritis induced by Freund's adjuvant, which allow evaluating the effects of potential agents in the treatment of arthritis for a prolonged period of time.

## Claims

1. A composition comprising aescin, at least one extract from a plant belonging to the genus *Boswellia* and one extract from *Scutellaria baicalensis.*

2. The composition according to claim 1, wherein aescin is present in the composition in an amount ranging from 0.5% to 70% by weight on the total weight of the composition, preferably in an amount ranging from 2 to 50% by weight on the total weight of the composition.

3. The composition according to any of claims 1 or 2, wherein the at least one extract from a plant belonging to the genus *Boswellia* is present in the composition in an amount ranging from 1 to 90% by weight on the total weight of the composition, preferably in an amount ranging from 5 to 80% by weight on the total weight of the composition.

4. The composition according to any one of claims 1 to 3, wherein the *Scutellaria baicalensis* extract is present in the composition in an amount ranging from 0.5 to 70% by weight on the total weight of the composition, preferably in an amount ranging from 2 to 50% by weight on the total weight of the composition.

5. The composition according to any one of claims 1 to 4, wherein the plant belonging to the genus *Boswellia* is *Boswellia serrata,*

6. The composition according to any one of claims 1 to 5, wherein aescin is present in the composition as an extract from *Aesculus hippocastanum.*

7. The composition according to any one of claims 1 to 6, which is a pharmaceutical composition, a dietary supplement, a medical device, a food for special medical purposes, a cosmetic for oral or topical administration.

8. The composition according to any one of claims 1 to 7, for use in the prevention and/or therapeutic treatment of osteoarticular diseases.

9. The composition for use according to claim 8, wherein the osteoarticular diseases are rheumatoid arthritis and/or osteoarthritis.

## Patentansprüche

1. Zusammensetzung, aufweisend Aescin, mindestens ein Extrakt aus einer Pflanze, die zur Gattung der *Boswelia* gehört, und ein Extrakt aus *Scutellaria baicalensis.*

2. Zusammensetzung nach Anspruch 1, wobei Aescin in der Zusammensetzung in einer Menge von 0,5 bis 70 Gew.-% bezogen auf das Gesamtgewicht der Zusammensetzung, bevorzugt in einer Menge von 2 bis 50 Gew.-% bezogen auf das Gesamtgewicht der Zusammensetzung enthalten ist.

3. Zusammensetzung nach einem der Ansprüche 1 oder 2, wobei das mindestens eine Extrakt aus einer Pflanze, die zur Gattung der *Boswelia* gehört, in der Zusammensetzung in einer Menge von 1 bis 90 Gew.-% bezogen auf das Gesamtgewicht der Zusammensetzung, bevorzugt in einer Menge von 5 bis 80 Gew.-% bezogen auf das Gesamtgewicht der Zusammensetzung enthalten ist.

4. Zusammensetzung nach einem der Ansprüche 1 bis 3, wobei das *Scutellaria bai*calensis-Extrakt in der Zusammensetzung in einer Menge von 0.5 bis 70 Gew.-% bezogen auf das Gesamtgewicht der Zusammensetzung, bevorzugt in einer Menge von 2 bis 50 Gew.-% bezogen auf das Gesamtgewicht der Zusammensetzung enthalten ist.

5. Zusammensetzung nach einem der Ansprüche 1 bis 4, wobei die zur Gattung der *Boswelia* gehörige Pflanze eine *Boswelia serrata* ist.

6. Zusammensetzung nach einem der Ansprüche 1 bis 5, wobei Aescin in der Zusammensetzung als Extrakt aus *Aesculus hippocastanum* enthalten ist.

7. Zusammensetzung nach einem der Ansprüche 1 bis 6, welche eine pharmazeutische Zusammensetzung, ein Nahrungsergänzungsmittel, ein Medizinprodukt, ein Nahrungsmittel für spezielle medizinische Zwecke, ein Kosmetikum zur oralen oder topischen Verabreichung ist.

8. Zusammensetzung nach einem der Ansprüche 1 bis 7 zur Verwendung in der Prävention und/oder der therapeutischen Behandlung osteoartikulärer Erkrankungen.

9. Zusammensetzung zur Verwendung nach Anspruch 8, wobei die osteoartikulären Erkrankungen rheumatoide Arthritis und/oder Osteoarthritis sind.

## Revendications

1. Composition comprenant de l'aescine, au moins un extrait d'une plante appartenant au genre *Boswellia* et un extrait de *Scutellaria baicalensis.*

2. Composition selon la revendication 1, dans laquelle l'aescine est présente dans la composition dans une quantité comprise dans la plage de 0,5 % à 70 % en poids, sur la base du poids total de la composition, de préférence dans une quantité comprise dans la plage de 2 à 50 % en poids, sur la base du poids total de la composition.

3. Composition selon l'une des revendications 1 ou 2, dans laquelle l'extrait d'une plante appartenant au genre *Boswellia,* au moins au nombre de un, est présent dans la composition dans une quantité comprise dans la plage de 1 à 90 % en poids, sur la base du poids total de la composition, de préférence dans une quantité comprise dans la plage de 5 à 80 % en poids, sur la base du poids total de la composition.

4. Composition selon l'une quelconque des revendications 1 à 3, dans laquelle l'extrait de *Scutellaria baicalensis* est présent dans la composition dans une quantité comprise dans la plage de 0,5 à 70 % en poids, sur la base du poids total de la composition, de préférence dans une quantité comprise dans la plage de 2 à 50 % en poids, sur la base du poids total de la composition.

5. Composition selon l'une quelconque des revendications 1 à 4, dans laquelle la plante appartenant au genre *Boswellia* est *Boswellia serrata,*

6. Composition selon l'une quelconque des revendications 1 à 5, dans laquelle l'aescine est présente dans la composition en tant qu'extrait *d'Aesculus hippocastanum.*

7. Composition selon l'une quelconque des revendications 1 à 6, qui est une composition pharmaceutique, un complément alimentaire, un dispositif médical, un aliment destiné à des fins médicales spéciales, un produit cosmétique destiné à une administration par voie orale ou topique.

8. Composition selon l'une quelconque des revendications 1 à 7, à utiliser dans la prévention et/ou le traitement thérapeutique de maladies ostéoarticulaires.

9. Composition à utiliser selon la revendication 8, dans laquelle les maladies ostéoarticulaires sont la polyarthrite rhumatoïde et/ou l'ostéoarthrite.
